# EUROPEAN PATENT SPECIFICATION

(11) **EP 4 061 460 B1**
(45) Date of publication and mention of the grant of the patent: **18.03.2026**
(21) Application number: 19953576.6
(22) Date of filing: 20.11.2019
(51) Int. Cl.: A61M 25/00, A61M 25/04, A61M 25/01

(54) **ISOLATION AND ATTACHMENT CATHETERS AND METHODS FOR USING THEM**
ISOLATIONS- UND BEFESTIGUNGSKATHETER UND VERFAHREN ZUR VERWENDUNG DAVON
CATHÉTERS D'ISOLEMENT ET DE FIXATION ET LEURS PROCÉDÉS D'UTILISATION

(43) Date of publication of application: 28.09.2022
(73) Proprietor: CLPH, LLC, Palo Alto, CA 94306 (US)
(72) Inventor: LEEFLANG, Stephen, Arie, Sunnyvale, CA 94086 (US); EVERSULL, Christian, Scott, Palo Alto, CA 94306 (US)
(74) Representative: Boult Wade Tennant LLP
(86) International application number: PCT/US2019/062503
(87) International publication number: WO 2021/101542

(56) References cited:
- WO-A1-2006/009856
- US-A1- 2005 261 667
- US-A1- 2008 214 889
- US-A1- 2015 173 592
- US-A1- 2017 119 956
- US-A1- 2017 224 283
- US-A1- 2017 224 283
- US-A1- 2018 036 514
- US-B2- 10 092 172

## Description

### FIELD OF THE INVENTION

The present invention relates generally to apparatus, systems, and methods for performing medical procedures, and, more particularly, to catheters adapted to attach to and/or isolate a portion of the heart wall during a procedure, e.g., during injection of one or more agents into the myocardium.

### BACKGROUND

Minimally invasive devices are frequently used to perform procedures within body lumens and chambers, including the heart. Such procedures include making injections of cells, agents, filler materials, and/or other substances into the myocardium by minimally invasive, e.g., catheter-based, approaches. During such procedures, it is important to identify the correct anatomical position for each injection, navigate to that position, and maintain the selected position while a needle or other injection device is introduced into the tissue, the injection is performed, and the injection device is subsequently removed. For example, heart motion may make precise needle positioning difficult. Further, unexpected or excessive movement of an exposed needle may cause trauma, such as laceration or perforation. Further, inadvertent or premature movement of the needle out of tissue may risk loss of injected material and/or embolic complications.

US 2008/214889 discloses methods and apparatus for preventing tissue migration or tissue "tenting" during transseptal access procedures. The underlying tissue wall may be temporarily engaged via any number of mechanisms, e.g., engaging teeth or projections, threaded needle, vacuum, etc., and one or more instruments may be passed through the tissue wall while maintaining engagement with the tissue to provide a counter-traction force. The procedure may be accomplished while under direct visualization within an imaging hood placed against the tissue surface and purged of blood.
US 2017/224283 discloses devices and systems for tissue engagement and methods of using the same. In at least one described device, the device comprises an elongated body having a proximal end, a distal end, and at least one lumen extending from the proximal end to the distal end; an engagement portion at the distal end of the elongated body, the engagement portion configured to engage a tissue adjacent thereto when the engagement portion contacts the tissue while suction is applied through the device; and at least one electrode present along the engagement portion and configured to contact the tissue when the engagement portion contacts the tissue and to obtain bioimpedance data from the tissue while suction is applied through the device

Thus, devices and methods that enhance stability of a catheter or other device and/or isolate the location of treatment during such procedures would be beneficial.

### SUMMARY

According to the present invention there is provided a system for injecting one of more agents into tissue within a patient's body as claimed in the appended claims.

The present invention is directed to apparatus, systems, and methods for performing minimally invasive medical procedures, e.g., within a patient's heart. More particularly, the present invention is directed to catheters including an attachment, stabilization, and/or isolation element that employs a vacuum source and which may be used during injection of agents, cells and/or other material into tissue including the myocardium or other tissue of the heart.

Catheters for performing injections may include an attachment, stabilization and or isolation element, e.g., employing suction/vacuum to attach to the wall of the heart. For example, an expandable tip, hood, cup or other element may be disposed at the distal end of the catheter to enable attachment or increased stability relative to the heart wall during a procedure. In the case of making injections into the wall of the heart, a number of unique challenges and risks arise, which may be addressed by one or more of the apparatus, systems, and methods described herein.

In one example useful for understanding the claimed invention, a catheter may include a vacuum hood including one or more radiopaque features adapted to clearly identify the heart wall, elucidate needle penetration into tissue, and/or demonstrate secure attachment of the vacuum hood to tissue. In another example, a catheter may include a vacuum hood adapted to enable needle penetration into tissue while preventing perforation of the heart wall. In yet another example, a catheter may include a vacuum hood adapted to generally reduce trauma to the vasculature, heart chamber, valves, and/or other tissue structures.

In accordance with another example, a telescoping catheter system may be provided that is adapted to enable clear identification of attachment to the heart wall.

In a further example, a catheter may include a vacuum hood adapted to enable oblique needle entry and securement into the heart wall.

In another example, a catheter may include a vacuum hood adapted to capture stray material before, during, or after injection into tissue.

In yet another example, a catheter system may be provided that includes a vacuum hood, vacuum lumen, and vacuum source adapted to minimize clotting and embolic potential of aspirated blood.

Other aspects and features of the present invention will become apparent from consideration of the following description taken in conjunction with the accompanying drawings.

### BRIEF DESCRIPTION OF THE DRAWINGS

The drawings illustrate exemplary embodiments of the invention, in which:
FIG. 1 is a side view of an exemplary embodiment of a catheter system including an outer catheter, a mid catheter with distally attached vacuum hood, and an inner injection catheter all slidable relative to one another, the system also including a vacuum source communicating with the lumen of the mid catheter and a source of one or more agents.
FIG. 2 is a cross-sectional side view of an exemplary embodiment of a vacuum hood with radiopaque features that may be provided on the hood and associated catheter, such as the mid catheter of FIG. 1.
FIGS. 3A and 3B are fluoroscopic images showing appearance of the radiopaque features of the vacuum hood of FIG. 2 before and after attachment, respectively, of the vacuum hood to a tissue wall.
FIGS. 4A and 4B show side views of another embodiment of a vacuum hood including fluoroscopic features, showing the vacuum hood before and after attachment, respectively, to a tissue wall.
FIG. 5 is a cross-sectional view of a heart, showing a catheter system with outer catheter, mid catheter, vacuum hood and injection catheter being manipulated within a chamber of the heart.
FIGS. 6A-6C show use of a vacuum hood to enable secure oblique entry of a needle into a tissue wall, e.g., using the system shown in FIGS. 1 and 5.
FIG. 7 is a side view of another exemplary embodiment of a mid catheter including a vacuum hood and a peripheral lumen for infusion/circulation of fluid within an interior chamber of the vacuum hood.
FIG. 7A is a cross-sectional view of the mid catheter of FIG. 7, taken along plane 7A-7A.

### DETAILED DESCRIPTION OF THE EXEMPLARY EMBODIMENTS

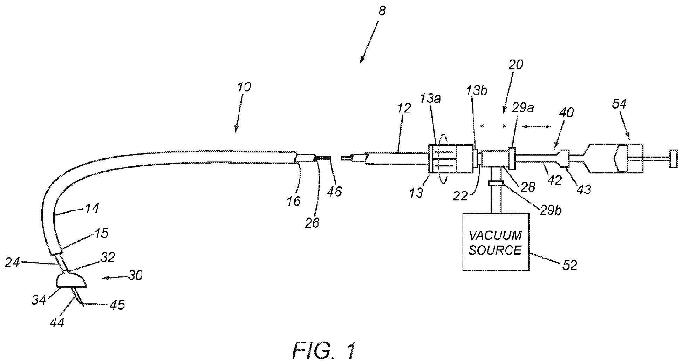

Turning to the drawings, FIG. 1 shows an exemplary embodiment of a catheter delivery system 8 including a first or outer catheter or sheath 10, a second or mid catheter 20 carrying a vacuum hood 30, and a third or injection catheter or needle device 40, which may be used to perform a procedure within a patient's body, e.g., perform one or more injections into tissue, such as within the myocardium of a patient's heart (not shown). In addition to the catheters 10, 20, 30, the system 8 may include one or more additional components, e.g., a vacuum source 52, such as a syringe or vacuum pump, and a source of one or more agents 54, e.g., including one or therapeutic and/or diagnostic materials, such as cells, filler materials, gels, and the like. Optionally, the system 8 may include one or more additional devices, e.g., a source of fluid, and/or one or more guidewires, sheaths, and the like (not shown) for facilitating introduction of the catheters into a patient's body.

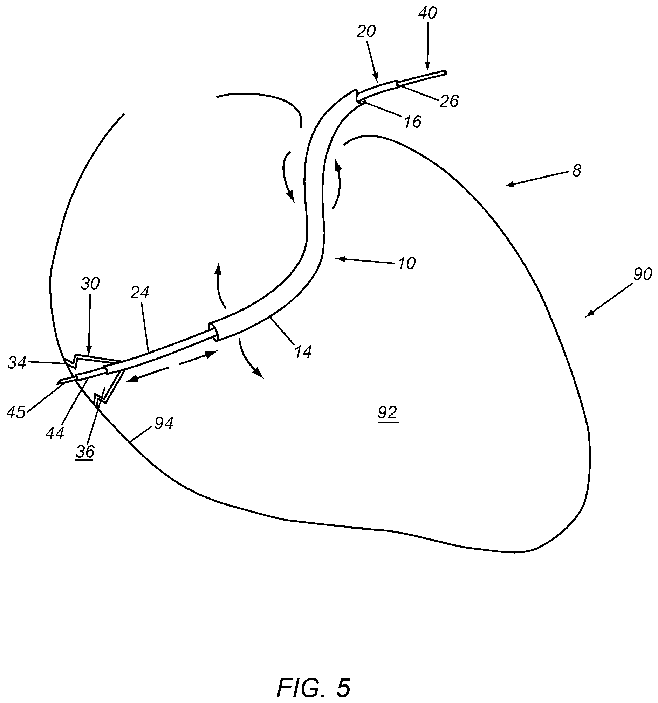

The outer catheter 10 generally includes a proximal end 12 including a hub or handle 13, a distal end 14 sized for introduction into a patient's body and terminating in a distal tip 15, and one or more lumens, e.g., lumen 16, extending between the proximal and distal ends 12, 14, thereby defining a longitudinal axis 18 for the catheter 10 and system 8. Optionally, a distal portion of the outer catheter 10 may be pre-shaped, deflectable, and/or otherwise directable to manipulate the distal end 14, e.g., to facilitate navigation within a patient's vasculature and/or chambers of the heart. For example, the outer catheter 10 may include one or more pull wires or other steering elements (not shown) coupled between the distal tip 15 and an actuator 13a on the handle 13, e.g., for directing the distal end 14 between a substantially straight and one or more curvilinear shapes, e.g., the shape shown in FIG. 5.

The outer catheter 10 may be constructed using known materials and methods, e.g., including a liner surrounding the lumen 16, an intermediate reinforcing layer (e.g., a braid, coil, and the like), and one or more outer jackets (all not shown for simplicity). In an exemplary embodiment, the liner may include one or more coatings, e.g., a hydrophobic, hydrophilic, or other lubricious coating, and/or may be formed from lubricious material to facilitate introduction and/or movement of the mid catheter 20 or other instrument within the lumen 16. Optionally, the outer catheter 10 may include one or more radiopaque markers, e.g., rings, bands, and the like, at desired locations along its length, e.g., at the distal tip 15 and/or spaced apart from one another adjacent the distal tip 15 (not shown).

In addition, the outer catheter 10 may include one or more additional components on the handle 13, e.g., a port 13b communicating with the lumen 16 and including one or more valves, e.g., a hemostatic valve (not shown) to provide a fluid-tight seal yet accommodate inserting the mid catheter 20 and/or other devices into the lumen 16. Optionally, the handle 13 may also include a side port including a Luer connector or other fitting (not shown) for coupling a source of fluid to the handle 13 and delivering the fluid into the lumen 16 (or an additional lumen, not shown) of the catheter 10. Exemplary embodiments of catheters that may be used as the outer catheter 10 are disclosed in U.S. Publication No. 2016/ 0082226.

With continued reference to FIG. 1, the mid catheter 20, similar to the outer catheter 10, generally includes a proximal end 22, a distal end 24, and one or more lumens, e.g., lumen 26, extending between the proximal and distal ends 22, 24. In addition, the mid catheter 20 includes an expandable vacuum hood 30 carried on the distal end 24 adjacent a distal tip 25 thereof, as described further elsewhere herein. The mid catheter 20 may be sized to be at least partially received within the outer catheter 10, e.g., having a diameter between about six and ten French (2.0-3.3 mm), or between about six and eight French (2.0-2.7 mm), such that the distal end 24 may be inserted through the port 13b in the handle 13 into the lumen 16 of the outer catheter 10, at any time during use, e.g., to deploy the vacuum hood 30, as described further elsewhere herein. Alternatively, the mid catheter 20 may be pre-loaded within and/or unremovable from the outer catheter 10, e.g., with the distal end 24 and vacuum hood 30 initially positioned within the lumen 16, e.g., proximal to the distal end 14 of the outer catheter 10. In another alternative, the hood 30 may be initially provided extending from the distal end 14 of the outer catheter 10, e.g., abutted against the distal tip 15, which may provide an atraumatic bumper during advancement of the outer catheter 10, e.g., through a valve or other tissue structure (not shown). In either case, the mid catheter 20 is configured to slide axially and/or rotationally relative to the outer catheter 10, e.g., to provide an additional degree of freedom when navigating within the vasculature or heart and/or to deploy and withdraw the vacuum hood 30 when desired from the outer catheter distal end 14, also as described elsewhere herein.

The mid catheter 20 may be constructed using similar materials and methods as the outer catheter 10, e.g., including a lubricious liner and/or coating, an intermediate reinforcement layer, and one or more outer jackets (not shown). Furthermore, the mid catheter 20 may include a relatively flexible distal segment, e.g., offset a predetermined distance proximal to the distal tip 25 and vacuum hood 30, to facilitate tracking and/or atraumatic navigation. For example, the flexible segment may be sufficiently flexible to stabilize the mid catheter 20, dampen movement, and/or prevent hood detachment, e.g., when the hood 30 is attached to a moving structure such as a wall of a beating heart (not shown), as described elsewhere herein.

The mid catheter 20 also includes a handle or hub 28 on the proximal end 24 including one or more ports, e.g., port 29a including one or more valves, e.g., a hemostatic valve (not shown) to provide a fluid-tight seal yet accommodate inserting the injection catheter 40 into the lumen 26. In addition, the handle 28 may also include a first side port 29b communicating with the lumen 26 of the mid catheter 20, which may include a Luer fitting or other connector (not shown) for coupling a vacuum source 52 to the side port 29b. Optionally, the handle 28 may include one or more additional side ports (not shown), e.g., a second side port communicating with a secondary lumen within the mid catheter 20 to which a source of fluid, e.g., a syringe (also not shown) may be coupled for delivering heparin, heparinized saline and/or other anticoagulant or dilutive media into the secondary lumen, as described elsewhere herein. Alternatively, or in addition, the mid catheter proximal end 24 may be fitted with a valve or hub including a valve, e.g., to seal on the body of a needle passing through the lumen 26 of the mid catheter 20, e.g., the body of the injection catheter 40 (not shown). A hole or passage (also not shown) may be provided in the side wall of the mid catheter 20, e.g., communicating with a lumen, e.g., the primary lumen 26 of the mid catheter 20, e.g., through which fluid may be infused or aspirated, e.g., to transmit negative pressure or vacuum to the vacuum hood 30.

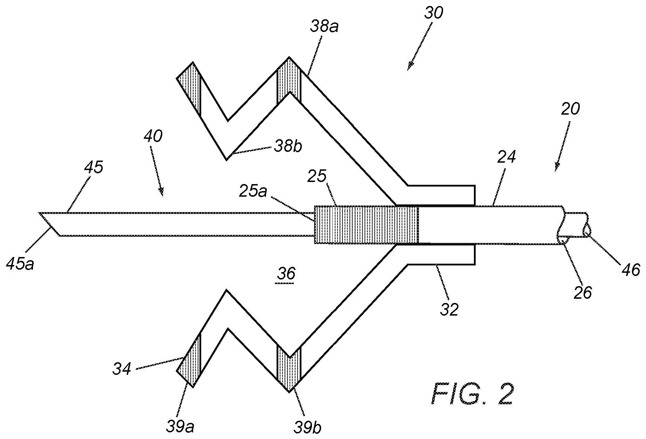

With additional reference to FIG. 2, a vacuum hood 30 is provided on the distal end 24 of the mid catheter 20 that includes an expandable element, e.g., a hood, cup, suction cup, sucker, and the like, which may be expandable between a collapsed configuration and an expanded configuration, e.g., having a diameter or other cross-sectional dimension larger than the outer diameter of the mid catheter 20 in the expanded configuration. In the embodiment shown in FIG. 2, the vacuum hood 30 includes a first or proximal end 32 that may be attached to the mid catheter 20 and a distal end or face 34 sized and/or shaped to be placed against a tissue surface (not shown), as described elsewhere herein. The vacuum hood 30 at least partially encloses an interior region or chamber 36 that is in communication with the primary lumen 26 of the mid catheter 20 and the distal face 34, e.g., such that vacuum may be generated within the chamber 36, and consequently at the distal face 34, via the primary lumen 26 from the vacuum source 52 coupled to the side port 29b. For example, the vacuum hood 30 may have a generally conical or frustoconical shape, a hemi-spherical, or other bulbous shape, e.g., that increases in size from the proximal end 32 to the distal face 34, e.g., generally symmetrical to the longitudinal axis 18.

In the collapsed configuration, the vacuum hood 30 may be rolled, folded, or otherwise compressed to a size sufficient to pass through the primary lumen 26, yet may be resiliently biased to assume the expanded configuration when deployed from the lumen 26. Optionally, the hood 30 may include one or more supports or features (not shown) formed into or attached to the hood material to bias the hood towards the expanded configuration and/or to bias the distal face 34 to open to a desired size and/or shape. For example, the folds or other features and/or markers may also provide support to the hood 30, e.g., to prevent the hood 30 from collapsing radially inwardly.

The vacuum hood 30 may be constructed of a generally flexible or compliant material, such as silicone, urethane, or polyether block amide, such that the vacuum hood 30 may be passed through and/or contact tissue structures, e.g., within a patient's vasculature and/or heart, without causing trauma, but having sufficient stiffness such that, when a vacuum is applied, the vacuum hood 30 does not entirely collapse. In particular, the vacuum hood 30 may be constructed with features to enable at least the distal face 34 to maintain a generally open shape with relatively consistent cross-sectional area, e.g., as described further elsewhere herein. It will be appreciated that the force with which the vacuum hood 20 is able to attach to tissue, e.g., to a wall of a heart, may be a function of both the vacuum applied and the cross-sectional area of the distal face 34 interfacing with the tissue. Thus, the force of attachment may be modulated by modulating these two parameters.

FIG. 2 shows a side view of an exemplary embodiment of the vacuum hood 30 attached to the distal end 24 of the mid catheter 20. In the embodiment shown, the proximal end 32 of the vacuum hood 30 may be attached to the distal end 24 proximal to a distal tip 25 of the mid catheter 20, i.e., such that the mid catheter distal tip 25 extends a predetermined distance into the chamber 36 of the vacuum hood 30. The vacuum hood 30 may be substantially permanently attached to the mid catheter distal end 24, e.g., by one or more of bonding with adhesive, interference fit, sonic welding, fusing, and the like.

Optionally, the vacuum hood 30 may include one or more annular gussets, folds, thin areas, thick areas, or other features 38 that support the hood 30 yet allow axial movement of the distal face 34 towards and/or away from the proximal end 32, e.g., to enable foreshortening of the hood 30 when vacuum is present within the chamber 36 of the hood 30, e.g., when attached to a wall of a heart or other tissue structure, as described further elsewhere herein. For example, the features 38 may simply be a thinning in the wall of the hood 30, more compliant material, a pleat, fold, bellows, or other mechanical feature, e.g., extending circumferentially around one or more locations along the length of the hood 30.

For example, in the embodiment shown in FIG. 2, the hood 30 may include an annular outward fold 38a and an annular inward fold 38b, thereby defining a bellows that allows the hood 30 to expand and contract axially, which may facilitate placement against a tissue structure, e.g., a wall of heart that is not oriented perpendicular to the longitudinal axis 18, as described further elsewhere herein. For example, the folds 38 may symmetrically compress or elongate, or may accommodate one side of the hood 30 elongating while an opposite side is compressed. Alternatively, multiple annular outward and/or inward folds may be provided, if desired, e.g., to increase the axial compression and/or elongation distance that the distal face 34 may be deflected relative to the mid catheter distal end 24.

In addition or alternatively, the hood 30 may include a flexible proximal region (not shown), e.g., spaced a predetermined distance from the proximal end 32, which may allow the distal face 34 of the hood 30 to pivot or gimbal relative to the longitudinal axis 18 and the proximal end 32 Such annular folds 38, features, and/or flexible regions may be molded or otherwise formed directly into the hood material or may be constructed by forming the hood 30 from multiple segments, e.g., annular segments, formed from different materials that are attached together.

The mid catheter distal tip 25 may include one or more outlet ports communicating with the primary lumen 26 of the mid catheter 20. For example, as shown in FIG. 2, the distal tip 25 includes an axial outlet 45a through which a needle tip 45 may extend during an injection. Optionally, the distal tip 25 may include one or more side ports (not shown) proximal to the outlet 45a, e.g., to facilitate a vacuum being applied to the chamber 36 when the needle tip 45 is positioned through the outlet 45a.

In addition, the mid catheter 20 and/or vacuum hood 30 may include one or more radiopaque markers to facilitate locating and/or positioning the distal end 24 and/or vacuum hood 30 using fluoroscopy or other imaging during a procedure, e.g., during introduction into and/or manipulation within a body lumen, placement of the hood distal face 34 against a tissue structure, and/or deployment of the injection catheter 40. For example, as shown in FIG. 2, the entire mid catheter distal tip 25 may be made from radiopaque material or may include one or more radiopaque rings, bands, or other discrete markers. Various methods may be used to make the distal tip 25 radiopaque, including doping of the outer jacket of the distal end 24 material within the chamber 36 with barium, bismuth, tungsten or other radiodense materials as known in the art. Alternatively, a separate annular tip may be formed from radiopaque material and attached to the mid catheter distal end 24. In another alternative, radiopaque bands or elements made of platinum, platinum/iridium, gold, tungsten or other radiodense metals may be provided on the distal tip 25, which may simply be an extension of the distal end 24, as is also known. Optionally, one or more radiopaque markers may be provided on the distal end 24 of the mid catheter body 20 proximal to the distal tip 25, if desired, e.g., using similar methods.

The extension of the mid catheter distal tip 25 into the chamber 36 of the vacuum hood 30 may provide structural support, preventing collapse of the hood 30 under vacuum, and/or may provide a fluoroscopically visible marker in known relation to the position of the vacuum hood 30 for navigation. For example, when vacuum is applied to the vacuum hood 30, a proximal segment adjacent the proximal end 32 may have a tendency to collapse; in fact, the hood 30 may be designed specifically such that the proximal segment collapses, e.g., in order for the entire hood 30 to be more easily sheathed, pulled into a mid catheter lumen 26, and/or otherwise constrained, e.g., for introduction into the body. If the proximal segment were to collapse fully under vacuum, fluid communication to the distal face 34 of the hood 30 may be interrupted and no attachment to tissue achieved. Extending the distal tip 25 of the mid catheter 20 at least partially through the chamber 36 of the hood 30 may ensure that the distal face 34 of the hood 30 remains in fluid communication with the vacuum source 52 when vacuum is applied via the lumen 26. In addition or alternatively, the extension of the mid catheter distal tip 25 into the chamber 36 of the vacuum hood 30 may prevent the vacuum hood 30 from crossing the path of the needle tip 45 and being penetrated by the needle tip 45, e.g., when the vacuum hood 30 is pressed against or encounters anatomy, e.g., a wall of the heart.

The vacuum hood 30 includes one or more radiopaque markers 39, which may be used independently or in conjunction with one or more radiopaque markers on the mid catheter 20, such as the distal tip 25. For example, as shown in FIG. 2, the vacuum hood 30 includes two annular radiopaque rings or markers 39a, 39b spaced apart axially from one another. The annular markers 39 may extend continuously around the circumference of the hood 30 or may extend discontinuously around the circumference, e.g., defining by a plurality of lines, dots, and the like that are spaced apart from one another but define a circumferential line. As shown, a first annular marker 39a may be located at or near the distal face 34 and a second annular marker 39b spaced a predetermined distance proximally from the first marker 39a, e.g., adjacent the outward fold 38a. The markers 39 may be created similar to the materials and methods described elsewhere herein, e.g., by doping and/or embedding or applying various radiodense materials to the hood material. In addition or alternatively, the entire hood 30 may be made at least partially radiopaque. In an exemplary embodiment, the vacuum hood 30 may be formed from silicone doped with approximately 20% barium sulfate and the markers 39 may be formed from of silicone doped with tungsten and applied to the hood 30, e.g., to distinguish the base material and markers 39 under fluoroscopic imaging.

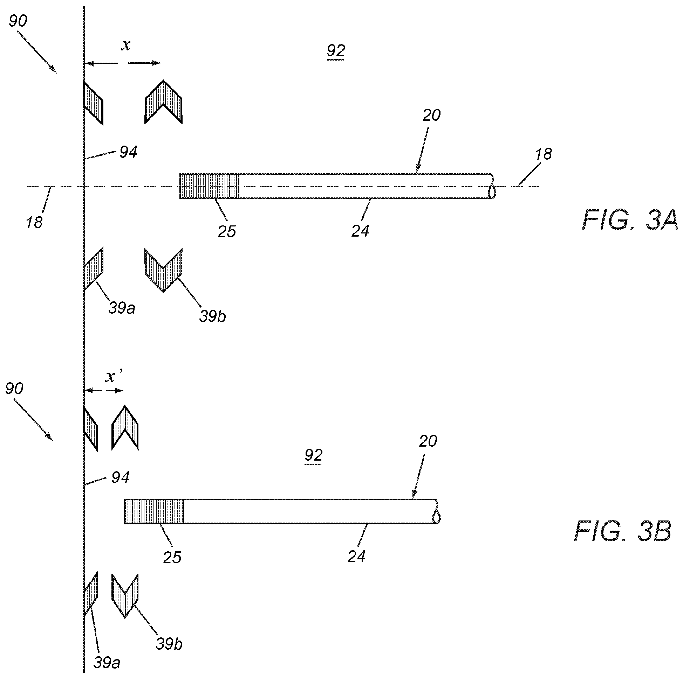

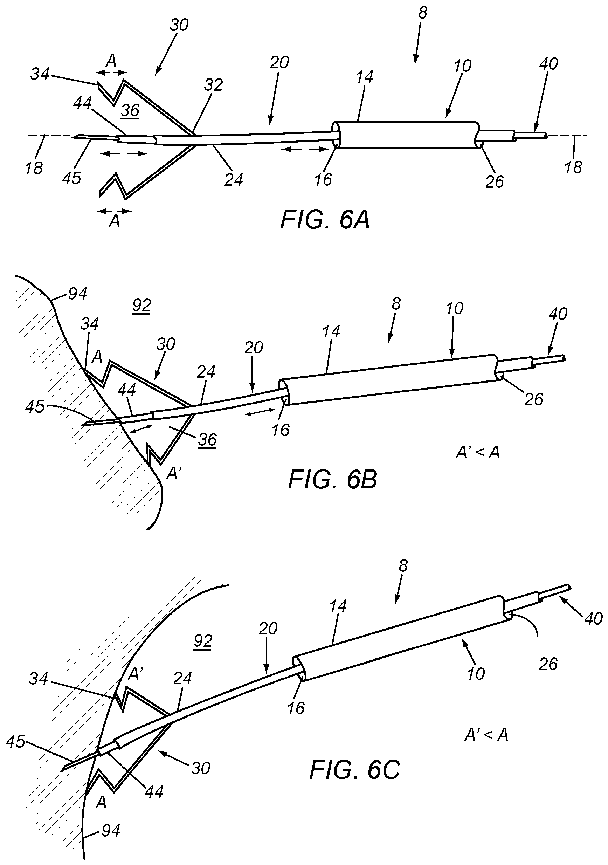

The markers 39 may facilitate positioning the vacuum hood 30 and/or confirm when the distal face 34 is seated and/or attached to a tissue structure, e.g., to a wall of a heart. For example, FIGS. 3A and 3B show exemplary fluoroscopic views of the vacuum hood 30 of FIG. 2, showing the relative position of the markers 39 before and after attachment of the distal face 34 to a wall 94 of a heart 90. FIG. 3 A shows a representative image that may be obtained when the vacuum hood 30 is in the expanded configuration, e.g., when deployed freely within a chamber 92 of a heart 90, e.g., as shown in FIG. 6A. In this configuration, the markers 39a, 39b are spaced apart from one another by a distance "X" corresponding to the distance between the markers 39a, 39b with the hood 30 in a relaxed configuration. During manipulation of the system 8 within the chamber 92, the distal face 34 of the vacuum hood 30 may initially come into contact with a tissue surface, e.g., the wall 94 of the heart 90, as shown in FIG. 3A. As the face 34 of the hood 30 is pressed against the wall 94 and a vacuum is applied within the chamber 36, the hood 30 may become attached and/or otherwise fixed to the wall 94 due to the vacuum, thereby compressing the hood 30 axially and directing the markers 39a, 39b closer together, e.g., to a smaller distance "X"', as shown in FIG. 3B. This new spacing may provide visual confirmation that the hood 30 is secured to the wall 94, whereupon an injection and/or other procedure may be performed, as described further elsewhere herein.

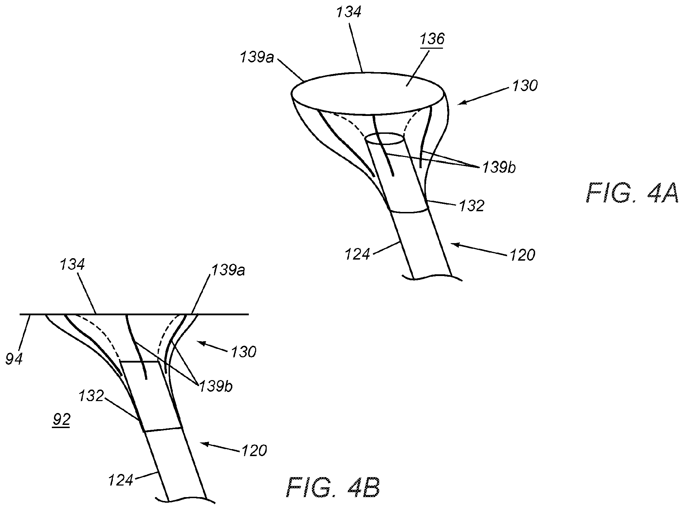

Alternatively, other configurations of markers may be provided on a vacuum hood to facilitate positioning and/or confirming when the hood is attached to a tissue structure. For example, FIGS. 4A and 4B shown another embodiment of a mid catheter 120 carrying a vacuum hood 130, which may be constructed generally similar to otherembodiments herein. However, in this embodiment, the hood 130 includes a first annular marker, e.g., a radiopaque ring 139a, provided at the distal face and two or more radiopaque stripes or bands 139b that extend at least partially along the length of the hood 130, i.e., partially between the proximal end 132 and the distal face 134. FIG. 4A shows the hood 130 in a relaxed or expanded configuration, e.g., when the hood 130 is deployed from an outer catheter (not shown), and the markers 139b extend generally axially towards the distal face 132. FIG. 4B shows the hood 130 in a compressed configuration, e.g., when pressed against a wall 94 within a chamber 92 of a heart and when vacuum is applied to attach the hood 130 to the wall 94. As shown, the radiopaque ring 139a may provide visual confirmation when the distal face 134 is pressed against the wall 93. In addition, the hood 130 may collapse partially radially inwardly when the vacuum is applied, thereby causing the markers 139b to move closer together, i.e., reducing the circumferential distance between adjacent markers 139b, all of which may be observed using fluoroscopy or other imaging to confirm the hood 130 has been successfully sealed and attached to the wall 94.

Returning to FIG. 1, the injection catheter 40 also includes a proximal end 42, a distal end 44, and at least one lumen 46 extending between the proximal and distal ends 42, 44, e.g., from a port in a handle or hub 43 to an outlet 45a in a distal tip 45, e.g., as shown in FIG. 2. The injection catheter 40 may be sufficiently flexible along its length, e.g., to enable advancement through the primary lumen 26 of the mid catheter 20 along a tortuous path created by the primary lumen 26, e.g., through a patient's vasculature from a percutaneous entry site into a chamber of a heart (not shown), as described elsewhere herein. The distal end 44 of the injection catheter 40 may terminate in a needle tip 45, e.g., having a size between 22 and 28 gauge, or between 26 and 28 gauge, and having a beveled, multi-faceted grind, trocar grind, or other sharpened shape, as desired to facilitate penetration into tissue.

Optionally, the distal end 44 of the injection catheter 40 may include a step down, e.g., a blunt surface from which the needle tip 45 extends, e.g., similar to the needle device shown in FIGS. 6A-6C, which may reduce the risk of inserting the needle tip 45 too far into tissue. In addition, as shown in FIG. 1, the handle 43 may include a port having one or more connectors, e.g., a Luer connector, for connecting a syringe or other source of injectable material 54 to the handle 43 for delivery into the needle lumen 46 and out the outlet 45a. Exemplary embodiments of needle devices that may be used for the injection catheter are disclosed in U.S. Publication No. 2017/ 0119156.

Generally, the system 8 of FIG. 1 may be used to navigate through a patient's body, e.g., through the patient's vasculature into a chamber of the patient's heart, to perform one or more injections using the injection catheter 40. For example, the system 8 may be used to access a left ventricle, e.g., using a retrograde aortic approach or an approach via the inter-atrial septum (not shown). Once in the heart, the vacuum hood 30 may be used to provide stability/attachment to the endocardial surface to facilitate introduction and maintenance of the needle tip 45 into the heart wall 94 to perform an injection. The vacuum hood 30 may be sized to accommodate typical topological features of the heart wall, for example, trabeculation in the ventricles, while maintaining a desired seal with the wall. In exemplary embodiments, the distal face 34 of the vacuum hood 30 may have a diameter in the expanded configuration between about four and fifteen millimeters (4-15 mm) or between about five and ten millimeters (5-10 mm). The combination of cross-section, material selection, mechanical design, and applied vacuum pressure may be adapted to provide sufficient attachment force in order to enable needle penetration, e.g., even without significant back support from the injection catheter 40.

For example, the attachment generated by the hood 30 and vacuum source54 may be between about 25-125 grams force or between about 50-100 grams force. Ideally, the attachment force may be high enough to enable penetration of the needle tip 45 into tissue, but low enough to prevent passage of the distal end 44 of the injection device 40 (proximal to the needle tip 45) through the heart wall, which may result in a perforation. Optionally, the injection catheter 40 may comprise a step up in diameter from the needle tip 45 to the distal end 45 of the injection catheter 40 (e.g., as shown in FIGS. 6A-6C) such that a lower force (e.g., <75 grams force) is required to introduce the needle tip 45 into the wall 94, but a higher force (e.g. >125 grams force) is required to perforate the wall 94 with the catheter body.

Turning now to FIG. 5, basic navigation of the system 8 within a chamber 92 of a heart 90, e.g., a left ventricle, is shown. The distal end 14 of the outer catheter 10 may be navigated into the chamber 92, e.g., from a percutaneous entry site (not shown) through any intervening body lumens. As described previously, optionally, a distal segment of the outer catheter 10 may have a predetermined or variable curve, e.g., biased to a predetermined curvilinear shape and/or steerable or otherwise deflectable from the proximal end 12 (not shown in FIG. 5; see FIG. 1). The outer catheter 10 may be advanced, retracted, rotated, and/or deflected, as desired, e.g., to provide alignment of its distal end 14 with a target location in the heart 90.

Once alignment is achieved, the mid catheter 20 may be advanced to direct the distal end 24 and hood 30 out of the lumen 16, e.g., until contact is made between the hood 30 and the heart wall 94 at the target location. For example, if the mid catheter 20 ]is pre-loaded into the lumen 16 with the hood 30 disposed adjacent the distal end 14, the mid catheter 20 may be advanced sufficiently to deploy the hood 30, which may resiliently expand to the expanded condition, e.g., as shown in FIG. 6A. Alternatively, if the mid catheter 20 is initially outside the outer catheter 10, the hood 30 may be directed to the collapsed configuration and the hood 30 and mid catheter 20 may be loaded into the outer catheter 10, e.g., through the port 29a into the lumen 16 (not shown; see FIG. 1) and then deployed from the outer catheter distal end 14 within the chamber 92. Alternatively, the hood 30 may start and remain deployed, e.g., positioned immediately adjacent the distal end 14 of the outer catheter 10, e.g., to provide a substantially atraumatic bumper, which may facilitate advancement of the outer catheter 10, e.g., through an aortic valve or other tissue structure along the path of introduction (not shown).

Contact of the hood distal face 34 with the wall 94 may be identified fluoroscopically, e.g., using the markers 39, 25 on the hood 30 and/or mid catheter 20, as described elsewhere herein. With the hood 30 in close proximity to and/or in direct contact with the wall 94, vacuum may be applied to the primary lumen 26 of the mid catheter 20 (by activating the vacuum source 52 shown in FIG. 1) and thus transmitted to the chamber 36 of the hood 30, facilitating attachment of the hood 30 to the heart wall 94.

For example, as the face 34 of the hood 30 comes into contact with the wall 94, the hood 30 may be displaced, bent, or otherwise moved relative to the distal tip 25 of the catheter 20, which may facilitate identification of the surface of the wall 94 under fluoroscopy. Further, the relative position of the hood markers 39 to the mid catheter distal tip 25 may change as the tissue surface is encountered. For example, in FIG. 6A, the distal tip 25 and hood markers 39 are shown in generally straight alignment, e.g., aligned concentrically with a central axis 18 of the system 8. However, when a surface is encountered, the markers 25, 39 may become misaligned due to the flexible connection between the hood 30 and mid catheter 20, e.g., if the hood 30 is deflected laterally relative to the distal end 24 of the hood, e.g., as shown in FIGS. 6B and 6C.

Optionally, the hood 30 may include one or more features that allow the hood 30 to conform to a tissue surface that is not orthogonal to the longitudinal axis 18 of the system 8. For example, the hood 30 may include one or more features enabling the hood 30 to bend relative to the longitudinal axis 18. In an exemplary embodiment, the hood 30 may include a mid segment fold, bend, bellows, or other feature enabling the hood 30 to compress on one side as tissue is encountered obliquely, e.g., as shown in FIGS. 6B and 6C. Alternatively, the proximal narrowed portion of the hood 30 may simply be sufficiently flexible to allow the distal face 34 of the hood 30 to turn towards the tissue as a tissue wall 94 is encountered. As previously described, the distal tip 25 of the mid catheter 20 may extend at least partially into the chamber 34 of the hood 30 to ensure transmission of vacuum to the distal face 34, e.g., even in the case of partial collapse of a proximal region of the hood 30. Thus, the vacuum hood 30 may attach to the tissue wall 94 to enable stable entry and maintenance of the needle tip 45 into tissue despite encountering the wall 94 at an angle or obliquely.

In addition or alternatively, in another option, a distal segment of the mid catheter 20, e.g., the segment extending beyond the outer catheter distal end 14 and the mid catheter distal tip 25, e.g., as shown in FIG. 5, may be formed from substantially flexible material such that, during or after attachment, heart wall motion may be accommodated without causing detachment of the hood 30 from the wall 94. Thus, in addition to transmitting vacuum and providing extension to navigate, the distal flexible segment may act as a suspension system or shock absorber between the outer catheter 10 and the hood 30, thereby facilitating stable attachment. Once the hood 30 is substantially attached to the heart wall 94, the needle tip 45 may be advanced into the wall 94 to injection one or more agents into the underlying tissue, e.g., as shown in FIG. 6B.

It will be appreciated that when vacuum is applied to the chamber 36 of the hood 30 and when the distal face 34 of the hood 30 forms a seal with the wall 94, the decrease in pressure within the chamber 34 may lead to at least partial collapse of the hood 30, or more specifically, to at least partial axial foreshortening of the hood 34, e.g., as shown in FIGS. 6B and 6C. In conjunction with such foreshortening, the radiopaque markers 39a, 39b may move closer together, as represented in the fluoroscopic image of FIG. 3B, or otherwise exhibit a change in special relationship that is readily appreciated fluoroscopically, thus providing a visual indicator of attachment of the hood 30 to the wall 94.

Thus, the radiopaque features 39, 25 of the hood 30 and/or the mid catheter 20 may be used to identify the location of the heart wall 94 and/or secure attachment of the hood 30 to the heart wall 94. Further, a periodic change in the relative position of the markers 39, 25 may be identified, e.g., due to contraction of the heart wall 94. In similar fashion, if the needle tip 45 is made at least partially radiopaque, the needle tip 45 may be visualized relative to other fluoroscopic markers of the system 8, e.g., the markers 39 and/or 25. Thus, the user may be able to verify that the needle tip 45 is extending a predetermined distance beyond, for example, the distal radiopaque marker 39a of the hood 30. In the attached state, this distal marker 39a may substantially delineate the wall 94 of the heart 90, such that the depth of penetration of the needle tip 45 into the wall 94 may be estimated based on the distance beyond the distal marker 39a. The injection device 40 may then be used to deliver one or more therapeutic and/or diagnostic materials, e.g., cells, agents, filler materials, gels, and/or other substances into the myocardium beyond the wall 94.

It will be appreciated that the hood 30 may provide a substantially isolated working area for performing injections and/or other procedures. This may be of particular importance where material to be injected provides a degree of embolic risk. For example, if an injection is made with a needle tip not fully engaged in the target tissue and/or if injected material leaks back out of the injection site and/or if injected material otherwise becomes free in the chamber 34, the hood 30 may isolate the free injected material and the vacuum source 52 may cause the free material to be aspirated through the lumen 26 of the mid catheter 20, rather than released into the chamber 92 of the heart 90.

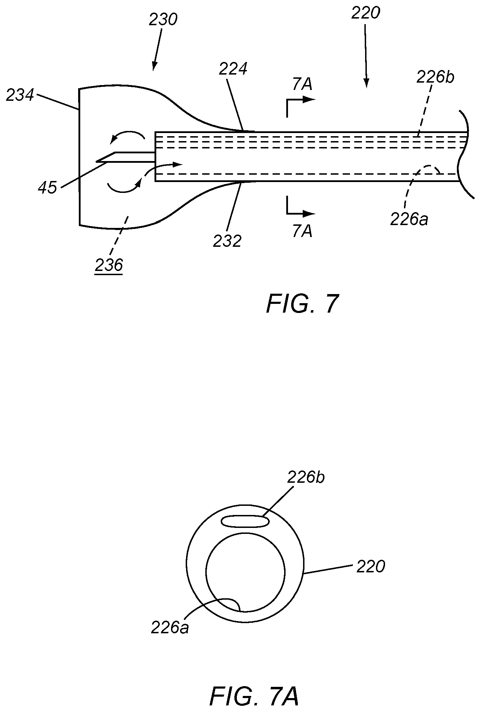

Turning to FIG 7, another embodiment of mid catheter 220 is shown that includes a vacuum hood 230 carried on a distal end 224 of the mid catheter 220. Similar to the previous embodiments, the mid catheter 220 includes a primary lumen 226a communicating with a chamber 234 of the hood 230, e.g., to apply a vacuum within the chamber 234 and distal face 234 to attach the hood 230 to a tissue structure (not shown). In addition, the mid catheter 220 includes a secondary or peripheral lumen 226b, e.g., offset from primary lumen as shown in FIG. 7A, adapted for infusion of fluid. As vacuum is applied to the mid catheter primary lumen 226a, blood may be drawn into the primary lumen 226a, e.g., before attachment of the distal face 234 to a tissue structure, in the case of partial attachment, and/or at the point of detachment from tissue. Blood drawn into the primary lumen 226a, if left static, may potentially clot and provide a possible source of embolic debris, e.g., as the injection device 40 is translated within the primary lumen 226a, or in the event that the lumen 226a is flushed. In addition or alternatively, a source of vacuum may be coupled to the secondary lumen 226b, e.g., to provide the vacuum to the chamber 234 and/or to augment the vacuum applied via the primary lumen 226a.

To avoid this situation, the peripheral lumen 226b may be used to infuse fluid, e.g., heparin, heparinized saline, and/or other anticoagulant or dilutive media into the chamber 236.. The fluid may be drawn back into the primary lumen 226a of the mid catheter 220 under vacuum and thereby diluting, clearing, and/or reducing the clotting potential of blood introduced into that lumen.

The foregoing disclosure of the exemplary embodiments has been presented for purposes of illustration and description. It is not intended to be exhaustive or to limit the invention to the precise forms disclosed. Many variations and modifications of the embodiments described herein will be apparent to one of ordinary skill in the art in light of the above disclosure.

Further, in describing representative embodiments, the specification may have presented the method and/or process as a particular sequence of steps. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the specification should not be construed as limitations on the claims.

While the invention is susceptible to various modifications, and alternative forms, specific examples thereof have been shown in the drawings and are herein described in detail. It should be understood, however, that the invention is not to be limited to the particular forms or methods disclosed, but to the contrary, the invention is to cover all modifications, equivalents and alternatives falling within the scope of the appended claims.

## Claims

1. A system (8) for injecting one or more agents into tissue within a patient's body, comprising:
a first tubular member (10) comprising a proximal end (12), a distal end (14) sized for introduction into a patient's body, and a lumen (16) extending between the proximal and distal ends;
a second tubular member (20) comprising a proximal end, a distal end (24) sized for introduction into a patient's body, and a lumen (26) extending between the proximal and distal ends, the second tubular member slidably disposed in the lumen of the first tubular member;
a needle catheter (40) slidably disposed in the lumen of the second tubular member;
a vacuum hood (30) attached to the distal end of the second tubular member; and
a vacuum source coupled to the second tubular proximal end for applying a vacuum through the second tubular member lumen to generate a vacuum within the vacuum hood,
the second tubular member comprising a radiopaque distal tip (25) extending at least partially into the vacuum hood, and
the vacuum hood having at least two radiopaque features (39a, 39b), the radiopaque features comprising first and second annular radiopaque markers (39a, 39b) spaced apart axially from one another, wherein when the vacuum hood is in a relaxed configuration the first and second annular radiopaque markers are a first distance (x) apart and when the vacuum hood is in a compressed configuration the first and second annular radiopaque markers are a second distance (x') apart such that the spatial relationship of the first and second annular radiopaque markers visibly changes under fluoroscopy when the vacuum hood is under sealed vacuum.

2. The system of claim 1, wherein the first and second annular markers (39a, 39b) extend continuously or discontinuously around a circumference of the vacuum hood.

3. The system of claim 1, wherein the vacuum hood comprises a proximal end (32) attached to the second tubular member distal end (24) proximal to the distal tip and a distal face (34) shaped for contacting a tissue surface within a patient's body.

4. The system of claim 3, wherein the vacuum hood includes one or more annular features (38) between the vacuum hood proximal end and the distal face to accommodate axial foreshortening and/or elongation of the vacuum hood.

5. The system of claim 3 or 4, wherein the second marker extends circumferentially around the vacuum hood at the distal face, such that the distance between the first and second markers provides the spatial relationship that visibly changes under fluoroscopy when the vacuum hood is under sealed vacuum.

6. The system of claim 3, wherein the vacuum hood includes one or more annular folds defining a bellows offset a predetermined distance proximal to the distal face to accommodate axial foreshortening and elongation of the vacuum hood.

7. The system of claim 3, wherein the vacuum hood includes an annular region offset a predetermined distance proximal to the distal face, the annular region comprising material that is more flexible than material immediately proximal and immediately distal to the annular region to provide a bendable region of the vacuum hood.

8. The system of claim 3, wherein the first annular marker extends circumferentially around the vacuum hood at the distal face.

9. The system of claim 8, further comprising a plurality of markers that extend at least partially axially between the vacuum hood proximal end and the distal face such that a circumferential distance between adjacent markers of the plurality of markers provides the spatial relationship that visibly changes under fluoroscopy when the vacuum hood is under sealed vacuum.

10. The system of claim 3, wherein the vacuum hood includes one or more annular features between the vacuum hood proximal end and the distal face to prevent collapse of the vacuum hood.

11. The system of claim 10, wherein one of the one or more annular features are doped with radiopaque material to define the first marker.

12. The system of claim 1, wherein the distal tip (25) of the second tubular member is configured to provide structural support, preventing collapse of the vacuum hood under vacuum, and/or provide a fluoroscopically visible marker in known relation to the position of the vacuum hood for navigation.

13. The system of claim 1, wherein distal tip (25) of the second tubular member is disposed at least partially through the chamber of the vacuum hood to ensure that the distal face of the vacuum hood remains in fluid communication with a vacuum source when vacuum is applied via the second tubular member lumen.

14. The system of any preceding claim, wherein the radiopaque distal tip (25) is formed by one of: doping the second tubular member distal end with barium, bismuth, tungsten or other radiodense materials; an annular tip formed from radiopaque material and attached to the second tubular member distal end; or radiopaque bands or elements made of platinum, platinum/iridium, gold, tungsten or other radiodense metals provided on the distal tip as an extension of the second tubular member distal end.

## Patentansprüche

1. System (8) zum Injizieren eines oder mehrerer Wirkstoffe in Gewebe im Körper eines Patienten, umfassend:
ein erstes rohrförmiges Element (10) mit einem proximalen Ende (12), einem distalen Ende (14), das zum Einführen in den Körper eines Patienten bemessen ist, und einem Lumen (16), das sich zwischen dem proximalen und dem distalen Ende erstreckt;
ein zweites rohrförmiges Element (20) mit einem proximalen Ende, einem distalen Ende (24), das zum Einführen in den Körper eines Patienten bemessen ist, und einem Lumen (26), das sich zwischen dem proximalen und dem distalen Ende erstreckt, wobei das zweite rohrförmige Element verschiebbar in dem Lumen des ersten rohrförmigen Elements angeordnet ist;
einen Nadelkatheter (40), der verschiebbar in dem Lumen des zweiten rohrförmigen Elements angeordnet ist;
eine Unterdruckhaube (30), die am distalen Ende des zweiten rohrförmigen Elements angebracht ist; und
eine Unterdruckquelle, die mit dem proximalen Ende des zweiten rohrförmigen Elements verbunden ist, um einen Unterdruck durch das Lumen des zweiten rohrförmigen Elements aufzubauen und einen Unterdruck in der Unterdruckhaube zu erzeugen,
wobei das zweite rohrförmige Element eine röntgendichte distale Spitze (25) umfasst, die sich zumindest teilweise in die Unterdruckhaube erstreckt, und
wobei die Unterdruckhaube mindestens zwei röntgendichte Merkmale (39a, 39b) aufweist, wobei die röntgendichten Merkmale eine erste und eine zweite ringförmige röntgendichte Markierung (39a, 39b) umfassen, die axial voneinander beabstandet sind, wobei, wenn sich die Unterdruckhaube in einer entspannten Konfiguration befindet, die erste und die zweite ringförmige röntgendichte Markierung einen ersten Abstand (x) voneinander haben, und wenn sich die Unterdruckhaube in einer komprimierten Konfiguration befindet, die erste und die zweite ringförmige röntgendichte Markierung einen zweiten Abstand (x') voneinander haben, so dass sich die räumliche Beziehung zwischen der ersten und der zweiten ringförmigen röntgendichten Markierung unter Fluoroskopie sichtbar ändert, wenn die Unterdruckhaube unter einem abgedichteten Unterdruck steht.

2. System nach Anspruch 1, bei dem sich die erste und zweite ringförmige Markierung (39a, 39b) kontinuierlich oder diskontinuierlich um einen Umfang der Unterdruckhaube erstrecken.

3. System nach Anspruch 1, bei dem die Unterdruckhaube ein proximales Ende (32), das am distalen Ende (24) des zweiten rohrförmigen Elements proximal zur distalen Spitze angebracht ist, und eine distale Fläche (34) umfasst, die geformt ist, um mit einer Gewebeoberfläche im Körper eines Patienten in Kontakt zu kommen.

4. System nach Anspruch 3, bei dem die Unterdruckhaube ein oder mehrere ringförmige Merkmale (38) zwischen dem proximalen Ende der Unterdruckhaube und der distalen Fläche aufweist, um eine axiale Verkürzung und/oder Verlängerung der Unterdruckhaube auszugleichen.

5. System nach Anspruch 3 oder 4, bei dem sich die zweite Markierung an der distalen Fläche in Umfangsrichtung um die Unterdruckhaube erstreckt, so dass der Abstand zwischen der ersten und der zweiten Markierung die räumliche Beziehung bildet, die sich unter Fluoroskopie sichtbar ändert, wenn die Unterdruckhaube unter einem abgedichteten Unterdruck steht.

6. System nach Anspruch 3, bei dem die Unterdruckhaube eine oder mehrere ringförmige Falten aufweist, die einen Balg definieren, der um einen vorgegebenen Abstand proximal zur distalen Fläche versetzt ist, um eine axiale Verkürzung und Verlängerung der Unterdruckhaube auszugleichen.

7. System nach Anspruch 3, bei dem die Unterdruckhaube einen ringförmigen Bereich umfasst, der um einen vorgegebenen Abstand proximal zur distalen Fläche versetzt ist, wobei der ringförmige Bereich ein Material umfasst, das flexibler ist als unmittelbar proximal und unmittelbar distal zum ringförmigen Bereich gelegenes Material, um einen biegsamen Bereich der Unterdruckhaube zu schaffen.

8. System nach Anspruch 3, bei dem sich die erste ringförmige Markierung auf der distalen Fläche in Umfangsrichtung um die Unterdruckhaube erstreckt.

9. System nach Anspruch 8, das ferner eine Mehrzahl von Markierungen umfasst, die sich zumindest teilweise axial zwischen dem proximalen Ende der Unterdruckhaube und der distalen Fläche erstrecken, sodass ein Umfangsabstand zwischen benachbarten Markierungen der Mehrzahl von Markierungen die räumliche Beziehung bereitstellt, die sich unter Fluoroskopie sichtbar ändert, wenn die Unterdruckhaube unter einem abgedichteten Unterdruck steht.

10. System nach Anspruch 3, bei dem die Unterdruckhaube ein oder mehrere ringförmige Merkmale zwischen dem proximalen Ende der Unterdruckhaube und der distalen Fläche aufweist, um ein Zusammenfallen der Unterdruckhaube zu verhindern.

11. System nach Anspruch 10, bei dem eines der ein oder mehreren ringförmigen Merkmale mit einem röntgendichten Material dotiert ist, um die erste Markierung zu definieren.

12. System nach Anspruch 1, bei dem die distale Spitze (25) des zweiten rohrförmigen Elements konfiguriert ist, um strukturelle Unterstützung zu bieten, die ein Zusammenfallen der Unterdruckhaube unter Unterdruck verhindert, und/oder einen fluoroskopisch sichtbaren Marker in bekannter Beziehung zur Position der Unterdruckhaube für die Navigation bereitzustellen.

13. System nach Anspruch 1, bei dem die distale Spitze (25) des zweiten rohrförmigen Elements zumindest teilweise durch die Kammer der Unterdruckhaube hindurch angeordnet ist, um sicherzustellen, dass die distale Fläche der Unterdruckhaube in Fluidverbindung mit einer Unterdruckquelle bleibt, wenn über das Lumen des zweiten rohrförmigen Elements Unterdruck angelegt ist.

14. System nach einem der vorstehenden Ansprüche, bei dem die röntgendichte distale Spitze (25) gebildet ist durch: Dotieren des distalen Endes des zweiten rohrförmigen Elements mit Barium, Wismut, Wolfram oder anderen röntgendichten Materialien; eine ringförmige Spitze, die aus röntgendichtem Material gebildet und am distalen Ende des zweiten rohrförmigen Elements angebracht ist, und/oder röntgendichte Bänder oder Elemente aus Platin, Platin/Iridium, Gold, Wolfram oder anderen röntgendichten Metallen, die an der distalen Spitze als Verlängerung des distalen Endes des zweiten rohrförmigen Elements vorgesehen sind.

## Revendications

1. Système (8) pour injecter un ou plusieurs agents dans un tissu du corps d'un patient, comprenant :
un premier élément tubulaire (10) comprenant une extrémité proximale (12), une extrémité distale (14) dimensionnée pour une introduction dans le corps d'un patient, et une lumière (16) s'étendant entre les extrémités proximale et distale ;
un second élément tubulaire (20) comprenant une extrémité proximale, une extrémité distale (24) dimensionnée pour une introduction dans le corps d'un patient, et une lumière (26) s'étendant entre les extrémités proximale et distale, le second élément tubulaire étant disposé de manière coulissante dans la lumière du premier élément tubulaire ;
un cathéter à aiguille (40) disposé de manière coulissante dans la lumière du second élément tubulaire ;
un capuchon d'aspiration (30) fixé à l'extrémité distale du second élément tubulaire ; et
une source de vide couplée à la seconde extrémité proximale tubulaire pour appliquer un vide à travers la lumière du second élément tubulaire afin de générer un vide à l'intérieur du capuchon d'aspiration, le second élément tubulaire comprenant une pointe distale radio-opaque (25) s'étendant au moins partiellement dans le capuchon d'aspiration, et le capuchon d'aspiration présentant au moins deux caractéristiques radio-opaques (39a, 39b), les caractéristiques radio-opaques comprenant des premier et second marqueurs radio-opaques annulaires (39a, 39b) espacés axialement l'un de l'autre, dans lequel lorsque le capuchon d'aspiration est dans une configuration détendue, les premier et second marqueurs radio-opaques annulaires sont à une première distance (x) l'un de l'autre et lorsque le capuchon d'aspiration est dans une configuration comprimée, les premier et second marqueurs radio-opaques annulaires sont à une seconde distance (x') l'un de l'autre de telle sorte que la relation spatiale des premier et second marqueurs radio-opaques annulaires change de manière visible sous fluoroscopie lorsque le capuchon d'aspiration est sous vide étanche.

2. Système selon la revendication 1, dans lequel les premier et second marqueurs annulaires (39a, 39b) s'étendent de manière continue ou discontinue autour d'une circonférence du capuchon d'aspiration.

3. Système selon la revendication 1, dans lequel le capuchon d'aspiration comprend une extrémité proximale (32) fixée à l'extrémité distale (24) du second élément tubulaire à proximité de la pointe distale et une face distale (34) mise en forme pour venir en contact avec une surface tissulaire à l'intérieur du corps d'un patient.

4. Système selon la revendication 3, dans lequel le capuchon d'aspiration inclut une ou plusieurs caractéristiques annulaires (38) entre l'extrémité proximale du capuchon d'aspiration et la face distale pour permettre un raccourcissement et/ou un allongement axial du capuchon d'aspiration.

5. Système selon la revendication 3 ou 4, dans lequel le second marqueur s'étend de manière circonférentielle autour du capuchon d'aspiration au niveau de la face distale, de sorte que la distance entre les premier et second marqueurs fournit la relation spatiale qui change de manière visible sous fluoroscopie lorsque le capuchon d'aspiration est sous vide étanche.

6. Système selon la revendication 3, dans lequel le capuchon d'aspiration inclut un ou plusieurs plis annulaires définissant un soufflet décalé d'une distance prédéterminée à proximité de la face distale pour permettre un raccourcissement et un allongement axial du capuchon d'aspiration.

7. Système selon la revendication 3, dans lequel le capuchon d'aspiration comprend une région annulaire décalée d'une distance prédéterminée à proximité de la face distale, la région annulaire comprenant un matériau qui est plus flexible que le matériau immédiatement proximal et immédiatement distal par rapport à la région annulaire pour fournir une région pliable du capuchon d'aspiration.

8. Système selon la revendication 3, dans lequel le premier marqueur annulaire s'étend de manière circonférentielle autour du capuchon d'aspiration au niveau de la face distale.

9. Système selon la revendication 8, comprenant en outre une pluralité de marqueurs qui s'étendent au moins partiellement axialement entre l'extrémité proximale du capuchon d'aspiration et la face distale de sorte qu'une distance circonférentielle entre des marqueurs adjacents de la pluralité de marqueurs fournit la relation spatiale qui change de manière visible sous fluoroscopie lorsque le capuchon d'aspiration est sous vide étanche.

10. Système selon la revendication 3, dans lequel le capuchon d'aspiration inclut une ou plusieurs caractéristiques annulaires entre l'extrémité proximale du capuchon d'aspiration et la face distale pour empêcher l'affaissement du capuchon d'aspiration.

11. Système selon la revendication 10, dans lequel une certaines des une ou plusieurs caractéristiques annulaires est dopée avec un matériau radio-opaque pour définir le premier marqueur.

12. Système selon la revendication 1, dans lequel la pointe distale (25) du second élément tubulaire est configurée pour fournir un support structurel, empêchant l'affaissement du capuchon d'aspiration sous vide, et/ou fournissant un marqueur visible par fluoroscopie en relation connue avec la position du capuchon d'aspiration pour une navigation.

13. Système selon la revendication 1, dans lequel la pointe distale (25) du second élément tubulaire est disposée au moins partiellement à travers la chambre du capuchon d'aspiration pour garantir que la face distale du capuchon d'aspiration reste en communication fluidique avec une source de vide lorsqu'un vide est appliqué via la lumière du second élément tubulaire.

14. Système selon l'une quelconque des revendications précédentes, dans lequel la pointe distale radio-opaque (25) est formée par un quelconque parmi :
un dopage de l'extrémité distale du second élément tubulaire avec du baryum, du bismuth, du tungstène ou d'autres matériaux radiodenses ;
une pointe annulaire formée d'un matériau radio-opaque et fixée à l'extrémité distale du second élément tubulaire ; ou
des bandes ou des éléments radio-opaques en platine, en platine/iridium, en or, en tungstène ou en d'autres métaux radiodenses fournis sur l'extrémité distale en tant que prolongement de l'extrémité distale du second élément tubulaire.
